# EUROPÄISCHE PATENTSCHRIFT

(11) **EP 1 624 896 B1**
(45) Veröffentlichungstag und Bekanntmachung des Hinweises auf die Patenterteilung: **29.07.2009**
(21) Anmeldenummer: 04733335.6
(22) Anmeldetag: 17.05.2004
(51) Int. Cl.: A61K 47/44, A61K 47/10

(54) **VERFAHREN ZUR HERSTELLUNG EINER STABILEN INJIZIERBAREN FORMULIERUNG VON PACLITAXEL**
METHOD FOR THE PRODUCTION OF A STABLE INJECTABLE FORMULATION MADE OF PACLITAXEL
PROCEDE DE PREPARATION D'UNE COMPOSITION INJECTABLE STABLE DE PACLITAXEL

(30) Priorität: 19.05.2003 AT 7602003
(43) Veröffentlichungstag der Anmeldung: 15.02.2006
(73) Patentinhaber: Ebewe Pharma Ges.m.b.H. Nfg. KG, 4866 Unterach (AT)
(72) Erfinder: EIDENHAMMER, Gerhard, A-5310 Mondsee (AT); SCHNAIT, Heinz, A-4863 Seewalchen (AT)
(74) Vertreter: Landgraf, Elvira
(86) Internationale Anmeldenummer: PCT/EP2004/005268
(87) Internationale Veröffentlichungsnummer: WO 2004/100994

(56) Entgegenhaltungen:
- EP-A- 0 645 145
- WO-A1-00/23070
- WO-A1-97/12017
- WO-A1-99/33780

## Beschreibung

Paclitaxel bzw. Taxol wird beispielsweise aus der Rinde der pazifischen Eibe, Taxus brevifolia, gewonnen und stellt ein mittlerweile etabliertes antineoplastisches Mittel dar.
Die Behandlung mit Paclitaxel wird beispielsweise bei Eierstockkrebs (vgl. u.a. McGuire et al. Ann. Int. Med., 111, 273-279), Brustkrebs (vgl. u. a. Holmes et al., Proceedings of the American Society of Clinical Oncology, Vol. 10, pp 60), Lungenkrebs (vgl. u.a. Brown et al., J of Clin Oncol, Vol. 9, No. 7 pp 1261 - 1267), Leukämie (vgl. u. a. Rowinsky et al., Cancer Research 49, 4640 - 4647) beschrieben.

Paclitaxel selbst ist in Wasser nur schwer löslich, daher ist zur Herstellung von beispielsweise injizierbaren Formulierungen von Paclitaxel, die Verwendung eines Lösungsvermittlers notwendig. Diese Formulierungen sollen auch mit wässrigen Infusionslösungen (z. B. 0,9% NaCl-Lösung, 5% Glukose) weiter verdünnt werden können. Paclitaxel ist aber in lipophilen Lösungsmitteln, beispielsweise Poly(oxyethylen)-Rizinusöl, wie Cremophor EL^{®} oder Cremophor ELP^{®}, sowie auch in alkoholischen Lösungsmitteln, wie beispielsweise Ethanol, nicht ausreichend stabil und wird bei Lagerung bei Raumtempatur zersetzt.

In derartigen Lösungen ist Paclitaxel nicht ausreichend stabil und wird zersetzt.

Aus der WO 94/12030 ist eine Formulierung von Paclitaxel in Poly(oxyethylen)-Rizinusöl (Cremophor) bekannt, die einen pH-Wert von kleiner 8.1, aufweist, der durch den Zusatz organischer oder anorganischer Säuren, bevorzugt durch Zusatz von Zitronensäure eingestellt wird.

In der EP 0 654 145 ist eine stabilisierte Formulierung einer pharmazeutischen Verbindung wie beispielsweise Paclitaxel beschrieben, die ein Lösungsmittel und ein nichtionisches Solubilisierungsmittel enthält, wobei die Formulierung einen derart gewählten Carboxylationengehalt aufweist, der niedrig genug ist um eine Zersetzung des Paclitaxel zu verhindern, wobei die Einstellung des pH-Werts unter 8.1 durch die Zugabe von HBr, HF oder HI erfolgt.

Aus WO 98/33780 sind Formulierungen von Paclitaxel mit gereinigtem Cremophor, der einen bestimmten maximalen Gehalt an mindestens einem bestimmten Kation enthält, bekannt.

Aus der WO 97/12017 ist ein Verfahren zur Reinigung von Cremophor bekannt, wobei zu Reinigung Aluminiumoxide oder Silikate verwendet werden. Derart gereinigte Cremophore werden zur Herstellung von Arzneimittelzubereitungen, insbesondere auch zur Herstellung von Paclitaxelformulierungen verwendet.

Bei all diesen Verfahren soll durch Behandlung des Solubilisierungsmittel und/oder durch Einstellung eines bestimmten pH-Wertes der Lösung von Paclitaxel eine ausreichende Stabilität der Formulierung gewährleistet werden.
Allerdings ist die Stabilität derartiger Formulierungen nach wie vor nicht befriedigend.

Aufgabe der Erfindung war es daher eine stabile injizierbare Formulierung von Paclitaxel bereitzustellen, die eine verbesserte Stabilität aufweist und auf einfache Weise herstellbar ist.

Gegenstand der Erfindung ist daher ein Verfahren zur Herstellung einer stabilen injizierbaren Formulierung von Paclitaxel, **dadurch gekennzeichnet, dass** eine Formulierung enthaltend Paclitaxel und ein Lösungsmittel und/oder Lösungsmittelsystem, das gegebenenfalls ein Solubilisierungsmittel enthält, mit einem Kationentauscher behandelt wird.

Die erfindungsgemäßen Formulierungen weisen eine verbesserte Stabilität auf.

Die Konzentration des Wirkstoffs in der Formulierung kann 1 - 10 mg/ml betragen, vorzugsweise 2 - 8 mg/ml, am meisten bevorzugt 6 mg/ml.

Als Lösungsmittel bzw. Lösungsmittelsysteme mit Solubilisierungsmittel kommen beispielsweise Ethanol, Ethanol/Poly(oxyethylen)-Rizinusöl, Ethanol/Polysorbat, Ethanol/Polyethyleneglycol und dergleichen in Frage, wobei die Mischungsverhältnisse je nach System zwischen 90:10 und 30:70 variieren können.
Im Lösungsmittelsystem Ethanol/Poly(oxyethylen)-Rizinusöl beträgt der Anteil an Ethanol vorzugsweise 90 - 50 Teile. Im Lösungsmittelsystem Ethanol/Polysorbat beträgt der Anteil an Ethanol vorzugsweise 40 - 60 Teile.

Der Wirkstoff wird in einem Lösungsmittel bzw. Lösungsmittelsystem gelöst und bei Raumtemperatur gerührt, bis das Paclitaxel vollständig gelöst ist. Anschließend wird die Lösung mit einem Kationentauscher behandelt.
Als Kationentauscher kommen beispielsweise Kationentauscher, die Sulfonsäuregruppen oder Carboxlatgruppen aufweisen, in Frage.
Verwendbare Kationentauscher können eine Matrix aus Polystyrol, Polystyrol Divinylbenzol Copolymer; Polyacrylester, Methacrylester Divinylbenzol Copolymer, Cellulose (z.B. Amberlite^{®}, Amberlyst^{®}, Dowex^{®}) enthalten. Die Aktivität beträgt typischerweise zwischen 2,0- 4,0 meq/g.
Die Menge des Kationentauschers beträgt vorzugsweise 0,01 - 10 % der Gesamtmenge des Ansatzes, vorzugsweise 0,05 - 1%. Die Behandlungsdauer beträgt typischerweise zwischen 2 und 24 Stunden.
Anschließend wird die so behandelte Lösung durch ein Filter entsprechender Porengröße, das eine vollständige Abtrennung des Kationentauschers erlaubt, filtriert und direkt in Vials abgefüllt.

Eine Vorreinigung des Lösungsmittels bzw. des Lösungsmittelssystems ist nicht erforderlich. Dadurch ist beispielsweise bei Verwendung eines Poly(oxyethylen)-Rizinusöl enthaltenden Lösungsmittelsystems keine zusätzliche Ausrüstung zur Destillation des Poly(oxyethylen)-Rizinusöl und/oder Filtration über Säulen vor oder nach der Behandlung mit einem Kationentauscher, notwendig.

Auf diese Weise hergestellte Formulierungen bzw. Vergleichslösungen wurden zur Untersuchung der Stabilität 2 Wochen bei 60°C gelagert. Anschließend wurde der Gehalt an Zersetzungsprodukten und der Wirkstoffgehalt der Lösung bestimmt.
Dabei zeigte sich, dass der Wirkstoffgehalt deutlich höher war als bei Vergleichsproben, die entweder nicht behandelt wurden oder durch Zusätze stabilisiert wurden. Ferner war der Gehalt an bekannten Abbauprodukte, sowie die Summe der Abbauprodukte deutlich geringer als bei den Vergleichsproben.

### Beispiele:

### Beispiel 1 und 2:

Poly(oxyethylen)-Rizinusöl ( 12,72 g) wird in Ethanol (87,82 g) gelöst und solange gerührt bis das gesamte Poly(oxyethylen)-Rizinusöl gelöst ist. Dann wird Paclitaxel mit einem Gehalt von 101,09 % (593.5 mg) in 100 ml dieser Lösung zugefügt und gerührt bis eine klare Lösung entstanden ist.
Die Lösung wird in zwei Teile aufgeteilt und ein Teil wird ohne Kationentauscherbehandlung über 0,2 µm Filter zu je 5 ml in sterilisierte Durchstichflaschen gefüllt, mit Stickstoff begast und mit einem sterilisierten Stopfen für Injektionslösungen verschlossen.
Der andere Teil der Lösung wird mit dem Kationentauscher (0,1g/100 g Lösung) behandelt. Die Lösung wird 16 Stunden geschüttelt. Diese Lösung wird über 0,2µm Filter zu je 5 ml in sterilisierte Durchstichflaschen gefüllt, mit Stickstoff begast und mit einem sterilisierten Stopfen für Injektionslösungen verschlossen. Ein Teil der Proben wird für die Ausgangsanalyse verwendet. Die restlichen Durchstichflaschen werden zwei Wochen 60°C gelagert und anschließend analysiert.

### Beispiel 3 und 4:

Poly(oxyethylen)-Rizinusöl ( 25,44 g) wird in Ethanol (78,06 g) gelöst und solange gerührt bis das gesamte Poly(oxyethylen)-Rizinusöl gelöst ist. Dann wird Paclitaxel mit einem Gehalt von 101,09 % (593.5 mg) in 100 ml dieser Lösung zugefügt und gerührt bis eine klare Lösung entstanden ist.
Die weitere Vorgangsweise erfolgt wie unter Beispiel 1 und 2 beschrieben.

### Beispiel 5 und 6:

Poly(oxyethylen)-Rizinusöl ( 38,16 g) wird in Ethanol (68,30 g) gelöst und solange gerührt bis das gesamte Poly(oxyethylen)-Rizinusöl gelöst ist. Dann wird Paclitaxel mit einem Gehalt von 101,09 % (593.5 mg) in 100 ml dieser Lösung zugefügt und gerührt bis eine klare Lösung entstanden ist.
Die weitere Vorgangsweise erfolgt wie unter Beispiel 1 und 2 beschrieben.

### Beispiel 7 und 8:

Polysorbat 80 ( 60 ml) wird zu Ethanol (60 ml) hinzugefügt und solange gerührt bis eine homogene Lösung entstanden ist. Dann wird Paclitaxel mit einem Gehalt von 101,09 % (593.5 mg) in 100 ml dieser Lösung zugefügt und gerührt bis eine klare Lösung entstanden ist.
Die weitere Vorgangsweise erfolgt wie unter Beispiel 1 und 2 beschrieben.

### Beispiel 9 und 10:

Paclitaxel mit einem Gehalt von 101,09 % (593.5 mg) wird in 100 ml Ethanol gerührt bis eine klare Lösung entstanden ist.
Die weitere Vorgangsweise erfolgt wie unter Beispiel 1 und 2 beschrieben.

### Beispiel 11 und 12:

Poly(oxyethylen)-Rizinusöl ( 56,52 g) wird in Ethanol (43,90 g) gelöst und solange gerührt bis das gesamte Poly(oxyethylen)-Rizinusöl gelöst ist. Dann wird Paclitaxel mit einem Gehalt von 101,09 % (593.5 mg) in 100 ml dieser Lösung zugefügt und gerührt bis eine klare Lösung entstanden ist.

### Beispiel 14:

Poly(oxyethylen)-Rizinusöl (56,52 g) wird in Ethanol (43,90 g) gelöst und solange gerührt bis das gesamte Poly(oxyethylen)-Rizinusöl gelöst ist. Dann wird Paclitaxel mit einem Gehalt von 101,09 % (593.5 mg) und wasserfreie Zitronensäure (200 mg) in 100 ml dieser Lösung zugefügt und gerührt bis eine klare Lösung entstanden ist.

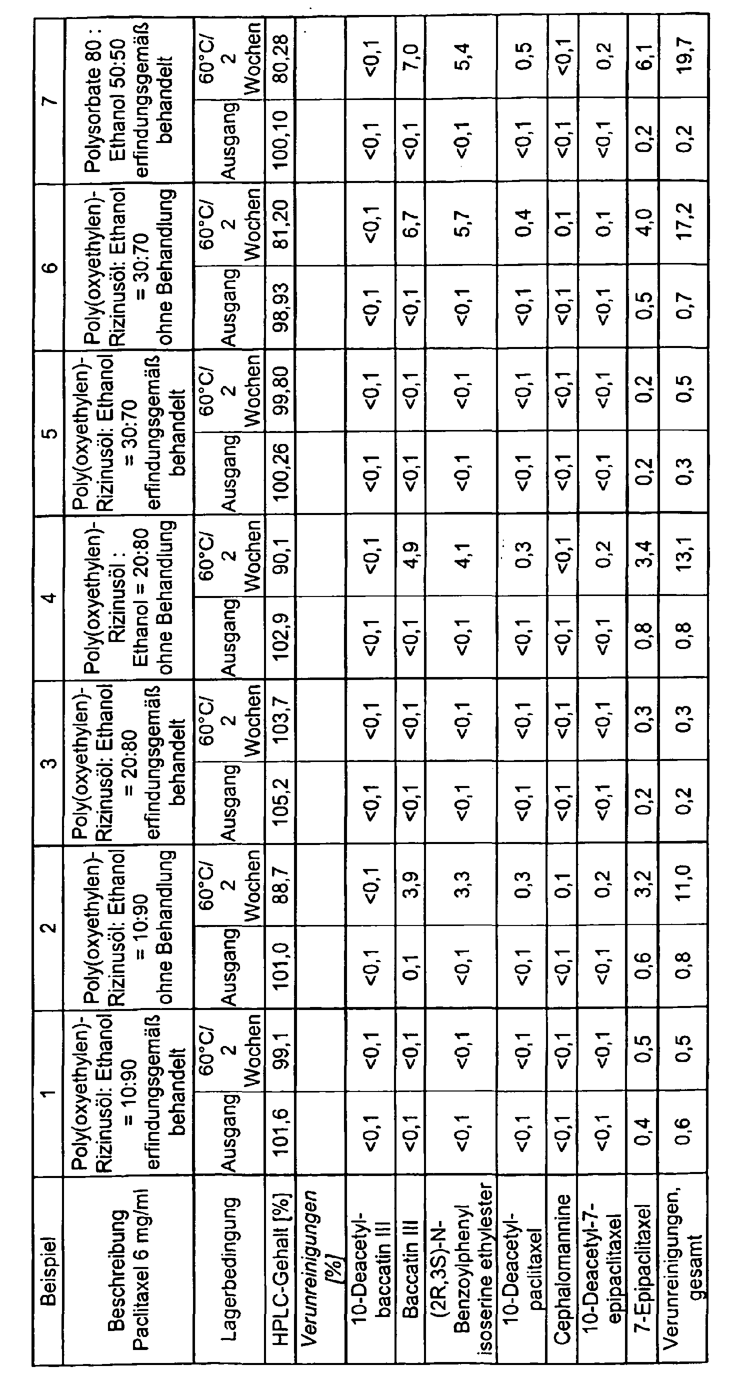

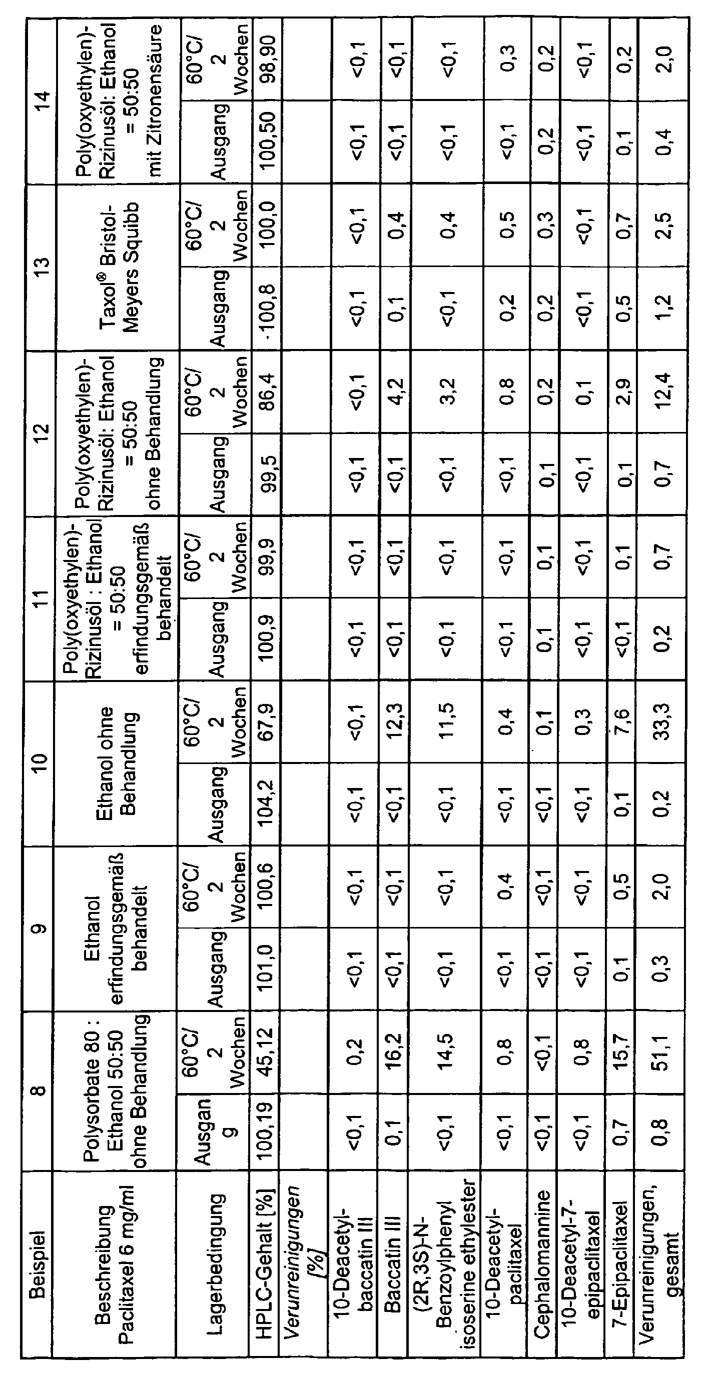

## Patentansprüche

1. Verfahren zur Herstellung einer stabilen injizierbaren Formulierung von Paclitaxel, **dadurch gekennzeichnet, dass** eine Formulierung enthaltend Paclitaxel und ein Lösungsmittel und/oder Lösungsmittelsystem, das gegebenenfalls ein Solubilisierungsmittel enthält, mit einem Kationentauscher behandelt wird.

2. Verfahren nach Anspruch 1, **dadurch gekennzeichnet, dass** der Wirkstoffgehalt in der Lösung 1-10 mg/ml beträgt.

3. Verfahren nach einem der Ansprüche 1 bis 2, **dadurch gekennzeichnet, dass** der Wirkstoffgehalt an Paclitaxel 4 - 8 g/ml beträgt, bevorzugt 6 mg/ml.

4. Verfahren nach einem der Ansprüche 1 - 3, **dadurch gekennzeichnet, dass** als Lösungsmittel bzw. Lösungsmittelsystem mit Solubilisierungsmittel Ethanol, Ethanol/Poly(oxyethylen)-Rizinusöl, Ethanol/Polysorbat, Ethanol/Polyethylene-glycol verwendet werden.

5. Verfahren nach Anspruch 4, **dadurch gekennzeichnet, dass** der Gehalt an Ethanol in einem Lösungsmittelsystem Ethanol/Poly(oxyethylen)-Rizinusöl 10 - 90 Teile beträgt.

6. Verfahren nach Anspruch 5, **dadurch gekennzeichnet, dass** der Gehalt an Ethanol in einem Lösungsmittelsystem Ethanol/Polysorbat 40 - 60 Teile beträgt.

7. Verfahren nach einem der Ansprüche 1 - 6, **dadurch gekennzeichnet, dass** als Kationentauscher ein Sulfonsäuregruppen oder Carboxylatgruppen enthaltender lonentauscher verwendet wird.

8. Verfahren nach einem der Ansprüche 1 - 7, **dadurch gekennzeichnet, dass** die eingesetzte Menge des Kationentauschers 0,01 - 10 % des Gesamtansatzes beträgt.

## Claims

1. A method of forming a stable injectable formulation of paclitaxel, **characterized in that** a formulation containing paclitaxel and a solvent and/or solvent system, which optionally contains a solubilizer, is treated with a cation exchanger.

2. A method according to Claim 1, **characterized in that** the active content of the solution is 1-10 mg/ml.

3. A method according to either of Claims 1 and 2, **characterized in that** the active content in terms of paclitaxel is 4-8 g/ml, preferably 6 mg/ml.

4. A method according to any one of Claims 1-3, **characterized in that** ethanol, ethanol/polyoxyethylene castor oil, ethanol/polysorbate, ethanol/polyethylene glycol are used as solvent or solvent system with solubilizer.

5. A method according to Claim 4, **characterized in that** the ethanol content of a solvent system ethanol/polyoxyethylene castor oil is 10-90 parts.

6. A method according to Claim 5, **characterized in that** the ethanol content of a solvent system ethanol/polysorbate is 40-60 parts.

7. A method according to any one of Claims 1-6, **characterized in that** an ion exchanger containing sulphonic acid groups or carboxylate groups is used as cation exchanger.

8. A method according to any one of Claims 1-7, **characterized in that** the amount of cation exchanger used is 0.01-10% of the total batch.

## Revendications

1. Procédé de préparation d'une composition injectable stable de Paclitaxel, **caractérisé en ce qu'**une composition comprenant du Paclitaxel et un solvant et / ou un système de solvants, qui contient le cas échéant un solubilisant, est traitée avec un échangeur de cations.

2. Procédé selon la revendication 1, **caractérisé en ce que** la teneur en principes actifs dans la solution est de 1 à 10 mg/ml.

3. Procédé selon une des revendications 1 à 2, **caractérisé en ce que** la teneur en principes actifs de Paclitaxel est de 4 à 8 g/ml, de préférence de 6 mg/ml.

4. Procédé selon une des revendications 1 à 3, **caractérisé en ce que** l'on utilise de l'éthanol, de l'éthanol / huile de ricin polyoxyéthylénée, de l'éthanol / polysorbate, de l'éthanol / polyéthylène glycol comme solvant ou système de solvants avec un solubilisant.

5. Procédé selon la revendication 4, **caractérisé en ce que** la teneur en éthanol dans un système de solvants éthanol / huile de ricin polyoxyéthylénée atteint 10 à 90 parties.

6. Procédé selon la revendication 5, **caractérisé en ce que** la teneur en éthanol dans un système de solvants éthanol / polysorbate atteint 40 à 60 parties.

7. Procédé selon une des revendications 1 à 6, **caractérisé en ce que** l'on utilise comme échangeur de cations un échangeur d'ions contenant des groupes d'acide sulfonique ou des groupes de carboxylate.

8. Procédé selon une des revendications 1 à 7, **caractérisé en ce que** la quantité mise en oeuvre de l'échangeur de cations atteint 0,01 à 10 % de la préparation totale.
